(19) Europäisches Patentamt
European Patent Office
Office européen des brevets

(11) Publication number: **0 176 238**
**A2**

(12) # EUROPEAN PATENT APPLICATION

(21) Application number: 85306005.1

(22) Date of filing: 23.08.85

(51) Int. Cl.⁴: C 07 C 67/04
C 07 C 69/14, C 07 C 69/54
C 07 C 69/24

(30) Priority: 29.08.84 GB 8421826

(43) Date of publication of application:
02.04.86 Bulletin 86/14

(84) Designated Contracting States:
BE DE FR GB IT NL

(71) Applicant: BP Chemicals Limited
Belgrave House 76 Buckingham Palace Road
London, SW1W 0SU(GB)

(72) Inventor: Green, Michael James
14 The Sett
Yateley Nr. Camberley Surrey(GB)

(74) Representative: Fawcett, Richard Fennelly et al,
BP INTERNATIONAL LIMITED Patents Division Chertsey Road
Sunbury-on-Thames Middlesex, TW16 7LN(GB)

(54) **Process for the preparation of glycol ether esters.**

(57) A process for the production of geminal glycol ether ester from an unsaturated ether and a carboxylic acid is provided. The process is carried out in the presence of, as catalyst, an effective amount of an acid insoluble in the reaction medium. Preferred acid catalysts include phosphoric acid and sulphonic acid resins.

EP 0 176 238 A2

PROCESS FOR THE PREPARATION OF GLYCOL ETHER ESTERS

The present invention relates to a process for the production of glycol ether esters and in particular to the preparation of geminal glycol ether esters from an unsaturated ether and a carboxylic acid.

The preparation of a geminal glycol ether ester from a vinyl ether and a carboxylic acid has been described in Chem. Abs. Volume 43 page 6576d and Chem Abs (1949) page 3785i. In the absence of catalyst reaction occurs but is slow. However the addition of a soluble mineral acid causes the reaction rate to accelerate rapidly.

It has now been discovered that solid insoluble acids can be used in effective amounts to catalyse the reaction between a vinyl ether and a carboxylic acid. An important feature of the catalysts described in this document is that they are both acidic and are insoluble in the reaction mixture. By using an insoluble acid catalyst for this reaction certain important advantages accrue. Firstly, by virtue of the fact that it is a solid, the catalyst is easily recoverable from the reaction products. Thus any simple mechanical method of separation can be used, for example filtration, separation by decanting and the like. Energy intensive separation procedures such as distillation can thus be avoided. Secondly, corrosion of the reaction vessels and associated plant is reduced. This is obviously important in cases where the plant is fabricated from a corrodable metal or alloy. Finally, by using a solid acid the process can be operated continuously using short residence lines. This is important as it avoids the production of significant

amounts of tar.

Accordingly, the present invention comprises a process for the production of a geminal glycol ether ester by the reaction of an unsaturated ether with a carboxylic acid, characterised in that the reaction is carried out in the presence of as catalyst a solid acid which is insoluble in the reaction mixture.

By the term geminal glycol ether ester is meant a compound having an ether group and an ester group attached to the same carbon atom as in for example the molecule 1-ethoxyethyl acetate formed by reaction of ethyl vinyl ether and acetic acid.

The unsaturated ether used can be any compound having the formula

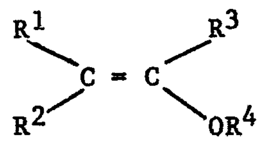

where $R^1$, $R^2$, $R^3$ and $R^4$ are independently aliphatic, alicyclic, heterocyclic or aromatic substituted or unsubstituted hydrocarbyl radicals. The hydrocarbyl radicals can themselves be substituted with further unsaturated ether groups. $R^1$, $R^2$ and $R^3$ can also be hydrogen or halide. It is preferred that $R^1$, $R^2$ and $R^3$ are either hydrogen or a $C_1$ to $C_{10}$ aliphatic hydrocarbyl radical and $R_4$ is a $C_1$ to $C_{10}$ aliphatic hydrocarbyl radical. Preferred examples are methyl vinyl ether, ethyl vinyl ether, propyl vinyl ether, butyl vinyl ether, methyl propenyl ether and the like.

The carboxylic acid used is suitably a lower alkyl carboxylic acid i.e. a $C_1$ to $C_{10}$ alkyl carboxylic acid, preferably acetic acid, propionic acid and the like. However, dicarboxylic acids e.g. maleic acid, succinic acid, tartaric acid and unsaturated acids e.g. acrylic acid, and methacryclic acid can also be used. The carboxylic acid can be either substituted or unsubstituted

Conveniently, the molar ratio of the two reactants is between 1:5 and 5:1, preferably between 1:2 and 2:1.

As regards the catalyst, this can be any acid which is insoluble in the reaction mixture but is suitably a phosphoric acid resin, e.g. Duolite ES467 or a sulphonic acid resin such as

Amberlite IRC50 or Amberlyst 15. The sulphonic acid resin can also be a fluorinated sulphonic acid resin. Typical inorganic acids, e.g. clays and zeolites can also be used. Conveniently the solid acid is added to the reaction mixture in amounts which constitute between 0.1 and 50% by weight of the total reaction mixture.

Although the reaction can be carried out at room temperature and pressure, the highly exothermic nature of the reaction means that it is usually necessary to cool the reaction medium externally in order to moderate it. It is desirable, if possible, to maintain the reaction mixture at a temperature in the range 20 to 80°C to achieve steady reaction and to reduce byproduct formation. After the reaction is complete, the reaction products are separated from the catalyst. If the process is carried out batchwise in the liquid phase the products can either be filtered or decanted off the catalyst. In this way the catalyst is available for immediate reuse.

If the process is operated continuously the reactants can be passed through a bed of the acid in an appropriate reactor.

The following Examples illustrate the invention described.

Example 1

10 g of Duolite ES467 ion exchange resin was treated with 30 ml of 1N hydrochloric acid to convert it to the acid form. 150 ml of deionised water was passed through the resin to remove any residual chloride ions followed by 150 ml of acetic acid to remove the water. The acetic acid saturated resin was transferred to a 150 ml 3-necked round-bottomed flask fitted with a reflux condenser, a thermocouple pocket, and an addition funnel. 36 g of ethyl vinyl ether was added rapidly, followed by 30 g of acetic acid. The resulting mixture was vigorously stirred for 1 hour, while the temperature in the flask was maintained at 28°C by external cooling. Analysis of the liquid product by gas chromatography showed a 49% conversion of ethyl vinyl ether with over 99% selectivity to 1-ethoxyethylacetate.

Comparative Example A

Example 1 was repeated in the absence of a catalyst. No

external cooling was required. Analysis of the liquid product showed a 26% conversion of ethyl vinyl ether to 1-ethoxyethylacetate.

Example 2

Example 1 was repeated, except that the reaction temperature was maintained at 40°C by external cooling. Analysis of the liquid product showed an 82% conversion of ethyl vinyl ether to 1-ethoxyethylacetate.

Example 3

Example 1 was repeated, except that the reaction temperature was maintained at 60°C by external cooling. Analysis of the liquid product showed a 96% conversion of ethyl vinyl ether to 1-ethoxyethylacetate.

Example 4

Example 2 was repeated except that the activated resin was washed with 150 ml of acrylic acid to remove the residual water and 36 g of acrylic acid was used in place of acetic acid. Analysis of the liquid product showed a 77% conversion of ethyl vinyl ether to 1-ethoxyethylacrylate.

Example 5

Example 1 was repeated using 0.5 g of Amberlyst 15 in place of Duolite ES467. The resin was supplied dry and in its acid form and no activation/drying was necessary. Analysis of the liquid product showed a 49% conversion of ethyl vinyl ether to 1-ethoxyethylacetate.

Example 6

Example 1 was repeated, except that 10 g of Amberlite IRC50 ion exchange resin was used in place of Duolite ES467. The resin is supplied in its acid form and no activation was necessary. Analysis of the liquid product showed a 45% conversion of ethyl vinyl ether to 1-ethoxyethylacetate.

Claims:

1. A process for the production of a geminal glycol ether ester by the reaction of an unsaturated ether with a carboxylic acid characterised in that the reaction is carried out in the presence of as catalyst a solid acid which is insoluble in the reaction mixture.

2. A process as claimed in Claim 1 characterised in that the solid acid is selected from a phosphoric acid resin, a sulphonic acid resin or a fluorinated sulphonic acid resin.

3. A process as claimed in Claim 1 characterised in that the carboxylic acid is a $C_1$ to $C_{10}$ alkyl carboxylic acid.

4. A process as claimed in Claim 1 characterised in that the unsaturated ether is one having the formula:

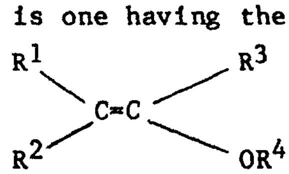

wherein $R^1$, $R^2$ and $R^3$ are either hydrogen or a $C_1$ to $C_{10}$ aliphatic hydrocarbyl radical and $R^4$ is a $C_1$ to $C_{10}$ aliphatic hydrocarbyl radical.

5. A process as claimed in Claim 4 characterised in that the unsaturated ether is selected from methyl vinyl ether, ethyl vinyl ether, propyl vinyl ether, butyl vinyl ether and methyl propenyl ether.